# FASCICULE DE BREVET EUROPEEN

(11) **EP 2 343 042 B1**
(45) Date de publication et mention de la délivrance du brevet: **18.10.2017**
(21) Numéro de dépôt: 10195541.7
(22) Date de dépôt: 17.12.2010
(51) Int. Cl.: A61K 8/58, A61K 8/46, A61K 8/81, A61K 8/73, A61Q 5/02, A61Q 5/12

(54) **Composition cosmétique comprenant au moins un compose organique du silicium, au moins un tensioactif anionique et au moins un polymère cationique**
Kosmetische Zusammensetzung, die mindestens eine organische Komponente aus Silizium, mindestens ein anionisches Tensid und mindestens ein kationisches Polymer enthält
Cosmetic composition containing at least one organic silicon compound, at least one anionic surface-active agent and at least one cationic polymer

(30) Priorité: 23.12.2009 FR 0959498
(43) Date de publication de la demande: 13.07.2011
(73) Titulaire: L'Oréal, 75008 Paris (FR)
(72) Inventeur: Viravau, Valérie, 75001, Paris (FR); Aires, Carine, 75014, Paris (FR); Drillon, Damien, 75008, Paris (FR)
(74) Mandataire: Dossmann, Gérard

(56) Documents cités:
- FR-A1- 2 413 907
- FR-A1- 2 749 507
- FR-A1- 2 789 896
- FR-A1- 2 814 363
- FR-A1- 2 930 439

## Description

La présente invention concerne une composition cosmétique pour le lavage et le conditionnement des fibres kératiniques, en particulier des fibres kératiniques humaines telles que les cheveux, comprenant, dans un milieu cosmétiquement acceptable, un ou plusieurs composés organiques du silicium, un ou plusieurs tensioactifs anioniques et un ou plusieurs polymères cationiques. La présente invention concerne également un procédé de traitement cosmétique des fibres kératiniques ainsi qu'une utilisation mettant en oeuvre ladite composition cosmétique.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux ou la peau.

Ces compositions de base présentent certes un bon pouvoir lavant mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (c'est-à-dire les cheveux qui se trouvent généralement abîmés ou fragilisés par l'action des agents atmosphériques extérieurs tels que la lumière et les intempéries, et/ou des traitements mécaniques ou chimiques tels que le brossage, le peignage, les teintures, les décolorations, les permanentes et/ou les défrisages), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents de conditionnement peuvent bien entendu également améliorer le comportement cosmétique des cheveux naturels.

Dans ce but, on a déjà proposé d'utiliser des composés organiques cosmétiquement actifs tels que des polymères cationiques et des silicones en tant qu'agents de conditionnement, dans des compositions cosmétiques détergentes tels que des shampooings, pour conférer aux cheveux des propriétés cosmétiques satisfaisantes, en particulier de la brillance, de la douceur, de la souplesse, de la légèreté, un toucher naturel ainsi qu'une aptitude au démêlage améliorée.

Cependant, l'emploi de ces composés dans des compositions cosmétiques de lavage et de conditionnement ne procure pas aux cheveux des propriétés coiffantes satisfaisantes et durables. En effet, ces compositions procurent généralement des effets coiffants, tels que des effets de maintien, de corps et/ou de discipline des cheveux, qui restent insuffisants et qui ont tendance à s'estomper après un lavage des cheveux avec un shampooing classique.

Or on a constaté que les consommateurs sont de plus en plus à la recherche de compositions de lavage qui soient non seulement capables de conditionner les cheveux de manière convenable mais également capables de procurer des effets coiffants satisfaisants et durables.

Ainsi des compositions destinées au lavage et au conditionnement des cheveux qui comprennent des composés organiques du silicium, tel que le 3-aminopropyltriéthoxysilane, ont été développées afin de pouvoir répondre à ces exigences. Ces compositions de lavage permettent de conditionner les cheveux, notamment en leur apportant un toucher doux satisfaisant, tout en conférant des effets coiffants marqués et durables.

De plus, ces compositions se sont révélées particulièrement avantageuses car elles permettent de faciliter la mise en forme des cheveux fins et de conférer des effets coiffants intéressants aux cheveux bouclés ou frisés, notamment en améliorant le dessin et le contrôle des boucles.

Toutefois, les compositions de lavage comprenant de tels composés organiques du silicium présentent généralement l'inconvénient d'évoluer sensiblement au cours du temps dans des conditions normales de stockage en fonction de la température, notamment au niveau de leur viscosité et de leur aspect visuel. En d'autres termes, ces compositions ne s'avèrent pas stables ce qui se traduit le plus souvent par un aspect visuel trouble ainsi que par une texture non satisfaisante au stockage.

En effet, il a été constaté que les composés organiques du silicium, tel que le 3-aminopropyltriéthoxysilane, n'étaient pas compatibles chimiquement avec la totalité des tensioactifs, notamment des tensioactifs anioniques, qui peuvent être présents dans les compositions de lavage, ce qui engendre les problèmes de stabilité rencontrés.

Par ailleurs, on a observé que l'introduction de certains composés organiques du silicium, en particulier les dérivés aminés tels que le 3-aminopropyltriéthoxysilane, dans des compositions de lavage, qui présentent généralement un pH allant de 4 à 7, engendre également des problèmes de stabilité du fait du caractère alcalin de ces composés.

Il existe donc un réel besoin de mettre au point des compositions cosmétiques destinées au nettoyage et au conditionnement des fibres kératiniques contenant des composés organiques du silicium, ces compositions ne présentant pas l'ensemble des inconvénients décrits ci-dessus, c'est-à-dire qui sont stables dans le temps et qui permettent de conditionner les cheveux de manière satisfaisante tout en apportant des effets coiffants puissants durables, notamment en termes de masse, de corps, de texturisation des cheveux.

La demanderesse a découvert, de façon surprenante, qu'il était possible de formuler des compositions de lavage et de conditionnement des fibres kératiniques, ayant les propriétés recherchées, comprenant un ou plusieurs composés organiques du silicium tels que définis ci-après, un ou plusieurs tensioactifs anioniques, un ou plusieurs polymères cationiques tels que définis ci-après et un ou plusieurs tensioactifs additionnels choisis parmi les tensioactifs amphotères et les tensioactifs non ioniques. En effet, il a été constaté que l'utilisation d'un ou plusieurs polymères cationiques tels que définis ci-après dans des compositions cosmétiques comprenant un ou plusieurs composés organiques du silicium et un ou plusieurs tensioactifs anioniques permet de les rendre stables au stockage aussi bien à température ambiante (20-25°C) qu'à 45°C, notamment au niveau de leur aspect visuel et de leur viscosité.

Par « stables » au sens de la présente invention, on entend que l'aspect visuel ainsi que la viscosité de ces compositions n'évoluent pas sensiblement au cours du temps dans des conditions standards de tests de stockage par exemple pendant les 2 mois à température ambiante (20°C-25°C), et/ou à 45°C et/ou à 4°C, qui suivent leur fabrication.

De plus, les compositions conformes à l'invention conduisent à un traitement satisfaisant des cheveux leur conférant ainsi un toucher doux satisfaisant, une aptitude au démêlage améliorée, de la douceur et de la souplesse.

Par ailleurs, les compositions conformes à la présente invention apportent des effets coiffants puissants, notamment en leur apportant de la masse, du corps et/ou de la discipline et ceci de manière durable.

De plus, les compositions selon l'invention permettent de faciliter la mise en forme des cheveux, en particulier des cheveux fins, et de conférer des effets coiffants améliorés aux cheveux bouclés, notamment en terme de dessin et de contrôle des boucles, et ceci de manière durable.

La présente invention concerne notamment une composition cosmétique pour le lavage et le conditionnement des fibres kératiniques, en particulier des fibres kératiniques humaines telles que les cheveux, comprenant dans un milieu cosmétiquement acceptable :
(i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques, et
(iii) un ou plusieurs polymères cationiques tels que définis ci-après, et
(iv) un ou plusieurs tensioactifs additionnels choisis parmi les tensioactifs amphotères et les tensioactifs non ioniques.
La présente invention concerne également un procédé de traitement cosmétique des cheveux, en particulier de lavage et de conditionnement, comprenant l'application sur lesdites fibres de la composition selon l'invention.

Elle concerne aussi l'utilisation de la composition selon l'invention comme shampoing pour le lavage et le conditionnement des cheveux.

D'autres objets et caractéristiques, aspects et avantages de l'invention apparaîtront encore plus clairement à la lecture de la description et des exemples qui suivent.

Les composés organiques du silicium (i) utilisés dans la composition selon l'invention sont choisis parmi les organosilanes comprenant un atome de silicium et les organosiloxanes comportant deux ou trois atomes de silicium, de préférence deux atomes de silicium. Ils doivent en outre comporter une ou plusieurs fonctions chimiques basiques, et de préférence une seule fonction chimique basique. La fonction chimique basique peut correspondre à toute fonction conférant un caractère basique au composé de silicium et est de préférence une fonction amine telle qu'une fonction amine primaire, secondaire ou tertiaire. Les composés du silicium selon l'invention, peuvent comporter éventuellement d'autres fonctions, telles que, par exemple, une fonction acide ou une fonction halogène.

Le ou les composés organiques du silicium (i) utilisés dans la composition selon invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les organosilanes utilisées dans la composition selon l'invention sont choisis parmi les composés de formule (I) : 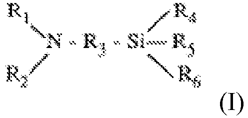 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R''', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, et deux au moins des groupes R₄, R₅ et R₆ désignant respectivement OR', OR" et OR''', deux au moins des groupes R', R" et R'" étant différents de l'hydrogène.

De préférence, les groupes R₁, R₂, R', R'₁, R'₂, R'₃, R" et R''' sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

Selon un autre mode de réalisation particulier, le ou les organosiloxanes utilisées dans la composition selon l'invention sont choisis parmi les composés de formule (II) : 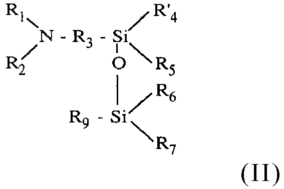 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ; R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR''', OR₁₀ ou OR₈.

De préférence, les groupes R"₁, R"₂, R₈ ou R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

En particulier, l'atome d'halogène est un atome de chlore.

Le ou les composés organiques du silicium utilisés dans la composition selon invention sont de préférence des organosilanes choisis parmi les composés de formule (III) : 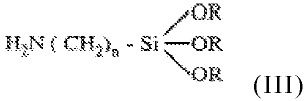 dans laquelle les radicaux R, identiques ou différents, sont choisis parmi les radicaux alkyle en C₁-C₆, de préférence en C₁-C₂, et n est un nombre entier de 1 à 6, de préférence de 2 à 4.

De préférence, les silanes ou les siloxanes sont solubles dans l'eau et encore plus préférentiellement solubles à la concentration de 2%, mieux à la concentration de 5% et encore mieux à la concentration de 10% en poids dans l'eau à la température de 25°C±5°C et à la pression atmosphérique. Par soluble, on entend la formation d'une phase macroscopique unique.

De façon particulièrement préférée, le composé organique du silicium (i) présent dans la composition selon l'invention est le 3-aminopropyltriéthoxysilane.

Le ou les composés organiques du silicium (i) peuvent être présents dans la composition selon l'invention dans une teneur allant de 0,01 à 10% en poids, de préférence dans une teneur en poids allant de 0,1 à 5% en poids, et plus préférentiellement dans une teneur en poids allant de 0,2 à 2% en poids, par rapport au poids total de la composition.

Comme indiqué précédemment, la composition cosmétique selon la présente invention contient en outre un ou plusieurs tensioactifs anioniques (ii).

Les tensioactifs anioniques (ii) utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50, mieux de 2 à 10 et encore mieux de 2 à 5 motifs oxyde d'éthylène, et leurs mélanges.

On utilise de préférence les alkylsulfates, les alkyléthersulfates, les acides alkyléther carboxyliques et leurs mélanges, en particulier sous forme de sels de métaux alcalins ou alcalino-terreux, d'ammonium, d'amine ou d'aminoalcool.

De préférence, le ou les tensioactifs anioniques (ii) utilisés dans la composition cosmétique selon l'invention sont choisis parmi les alkyléthersulfates, de préférence en C₁₂-C₁₄, et plus particulièrement comprenant de 2 à 3 moles d'oxyde d'éthylène.

Le ou les tensioactifs anioniques (ii) peuvent être présents dans les compositions cosmétiques selon l'invention dans une teneur allant de 1 à 25% en poids, de préférence dans une teneur allant de 3 à 20% en poids, et encore plus préférentiellement dans une teneur allant de 5 à 15% en poids, par rapport au poids total de la composition cosmétique selon l'invention.

De préférence, le ou les tensioactifs anioniques (ii) peuvent être présents dans les compositions cosmétiques selon l'invention dans une teneur supérieure à au moins 4% en poids par rapport au poids total de la composition cosmétique.

Comme indiqué précédemment, la composition cosmétique selon l'invention comprend en outre un ou plusieurs polymères cationiques (iii).

Au sens de la présente invention, l'expression "polymère cationique" désigne tout polymère contenant des groupements cationiques et/ou des groupements ionisables en groupements cationiques.

Les polymères cationiques (iii) présents dans la composition selon l'invention sont choisis parmi les celluloses cationiques et les gommes de guar cationiques, et en particulier parmi:
(2) Les dérivés d'éthers de cellulose comportant des groupements ammonium quaternaire décrits dans le brevet français 1 492 597, et en particulier les polymères commercialisés sous les dénominations "UCARE POLYMER JR" (JR 400, JR 125, JR 30M) ou "LR" (LR 400, LR 30M) par la société AMERCHOL. Ces polymères sont également définis dans le dictionnaire CTFA comme des ammonium quaternaires d'hydroxyéthylcellulose ayant réagi avec un époxyde substitué par un groupement triméthylammonium .
(3)Les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les produits commercialisés répondant à cette définition sont plus particulièrement les produits vendus sous la dénomination "Celquat L 200" et "Celquat H 100" par la Société National Starch.
(4) Les gommes de guar cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR C13 S, JAGUAR C 15, JAGUAR C 17 ou JAGUAR C162 par la société RHODIA.

Plus préférentiellement, le ou les polymères cationiques utilisés dans la composition cosmétique selon l'invention sont choisis parmi les hydroxyalkylcelluloses, comme les hydroxyméthyl-, hydroxyéthyl- ou hydroxypropyl celluloses greffées notamment avec un sel de méthacryloyléthyltriméthylammonium, de méthacrylamidopropyltriméthylammonium, de diméthyldiallyl ammonium, et les gommes de guar cationiques. Encore plus préférentiellement, le ou les polymères cationiques utilisés dans la composition cosmétique selon l'invention sont choisis parmi les celluloses cationiques.

La teneur en polymère(s) cationique(s) dans la composition selon l'invention peut varier de 0,01 à 5% en poids par rapport au poids total de la composition, de préférence de 0,1% à 1% en poids, et plus préférentiellement de 0,15% à 0,5% en poids, par rapport au poids total de la composition.

De préférence, le rapport pondéral entre le ou les composés organiques du silicium et le ou les polymères cationiques varie de 0,1 à 20, plus préférentiellement de 0,5 à 10 dans les compositions cosmétiques selon l'invention.

Encore plus préférentiellement, le rapport pondéral entre le ou les composés organiques du silicium de formule (III) et le ou les celluloses cationiques, en particulier les hydroxyalkylcelluloses cationiques, varie de 0,1 à 20

La composition selon la présente invention comprend également un ou plusieurs tensioactifs additionnels choisis parmi les tensioactifs amphotères et les tensioactifs non ioniques.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (XIII) et (XIV) :

Rₐ-CONHCH₂CH₂-N(R_{b})(R_{c})(CH₂COO⁻) (XIII)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH₂CH₂-N(B)(B') (XIV)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques varie de préférence dans l'intervalle allant de 0,1 à 15 % en poids, mieux encore de 0,5 à 10% en poids, encore mieux de 1 à 8% en poids, par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques additionnels varie de préférence de 0,01 à 20% en poids, mieux encore de 0,1 à 10 % en poids par rapport au poids total de la composition.

De préférence, la composition cosmétique comprend un ou plusieurs tensioactifs amphotères en tant que tensioactifs additionnels.

De préférence, la quantité totale en tensioactifs dans la composition cosmétique selon l'invention varie de 3 à 50% en poids, plus préférentiellement de 5 à 30% en poids, mieux de 8 à 20% en poids, par rapport au poids total de la composition cosmétique.

La composition cosmétique selon l'invention peut également comprendre un ou plusieurs acides organiques.

Par acide organique, on entend tout composé organique non polymérique comportant une ou plusieurs fonctions acides choisies parmi les fonctions acide carboxylique, acide sulfonique, acide phosphorique.

Préférentiellement, l'acide organique n'est pas un tensioactif.

Encore plus préférentiellement, le poids moléculaire de l'acide organique est inférieur à 250, mieux inférieur à 200.

Les acides organiques peuvent être des acides aminés.

Le ou les acides organiques sont choisis de préférence parmi l'acide acétique, l'acide propanoïque, l'acide butanoïque, l'acide lactique, l'acide malique, l'acide glycolique, l'acide ascorbique, l'acide maléïque, l'acide phtalique, l'acide succinique, la taurine, l'acide tartrique, l'arginine, la glycine, l'acide glucuronique, l'acide gluconique et l'acide citrique.

Encore plus préférentiellement, les acides organiques selon l'invention sont les acides carboxyliques et mieux les acides carboxyliques alpha-hydroxylés ou AHA.

Encore plus préférentiellement encore, l'acide organique utilisé dans la composition selon l'invention est l'acide lactique et l'acide citrique et de préférence l'acide lactique.

Dans la composition, l'acide organique peut être sous forme libre ou salifiée.

Le ou les acides organiques pouvant être utilisés dans la composition selon la présente invention peuvent être présents en une teneur exprimée en acides libres allant de 0,01 à 10% en poids, de préférence en une teneur allant de 0,1 à 8% en poids, et encore plus préférentiellement en une teneur allant de 0,2 à 5% en poids, par rapport au poids total de la composition.

La composition cosmétique selon l'invention peut également comprendre une ou plusieurs silicones, de préférence aminées.

Par « silicone aminée », on entend, au sens de la présente invention, toute silicone comportant au moins une fonction amine primaire, secondaire ou tertiaire ou un groupement ammonium quaternaire.

Les silicones aminées éventuellement utilisées dans la composition cosmétique selon la présente invention sont choisies parmi :
(a) les composés répondant à la formule (XV) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (XV)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :
      - N(R²)-CH₂-CH₂-N(R²)₂ ;
      - N(R²)₂ ; -N⁺(R²)₃ Q⁻ ;
      - N⁺(R²) (H)₂ Q⁻ ;
      - N⁺(R²)₂HQ⁻ ;
      - N(R²)-CH₂-CH₂-N⁺(R²)(H)₂ Q⁻,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (XV) sont choisies parmi les composés correspondant à la formule suivante : 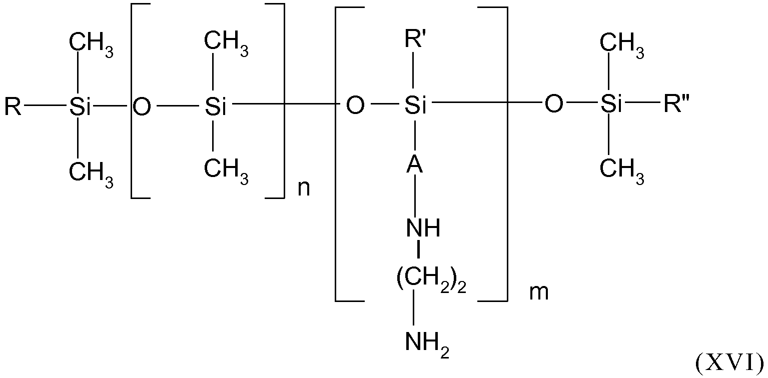 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (XV) est en particulier le polymère dénommé dans le dictionnaire CTFA "triméthylsilylamodiméthicone", répondant à la formule (XVIII) suivante: 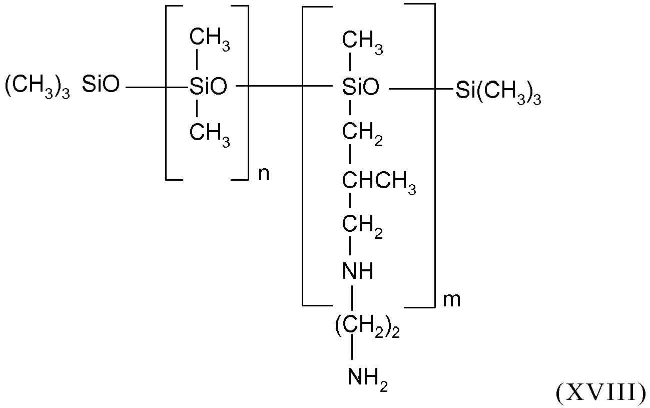 dans laquelle n et m ont les significations données ci-dessus conformément à la formule (XV).

De tels composés sont décrits par exemple dans EP 95238; un composé de formule (XVIII) est par exemple vendu sous la dénomination Q2-8220 par la société OSI.
(b) les composés répondant à la formule (XIX) suivante : 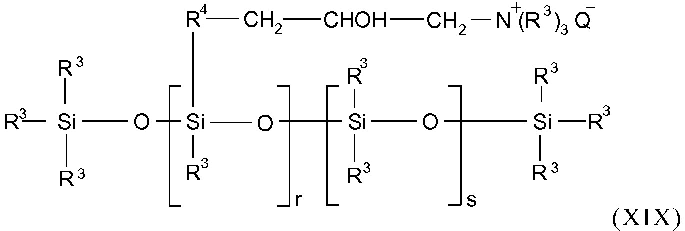 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US 4185087.

Un composé entrant dans cette classe est celui vendu par la Société Union Carbide sous la dénomination "Ucar Silicone ALE 56".
c) les silicones ammonium quaternaire de formule (XX) : 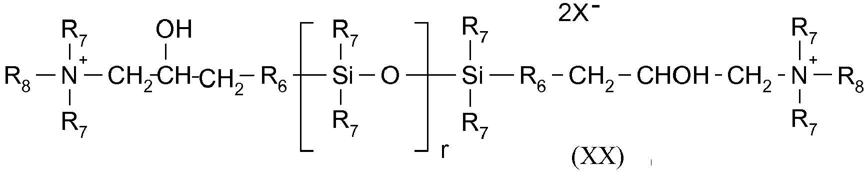 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC ;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

Ces silicones sont par exemple décrites dans la demande EP-A-0530974.
d) les silicones aminées de formule (XXI) : 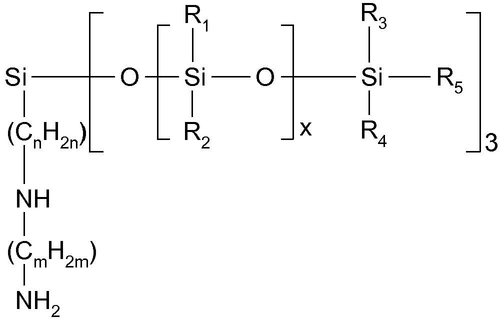 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
et dans laquelle x est choisi de manière telle que l'indice d'amine soit compris entre 0,01 et 1 meq/g.

De préférence, les silicones aminées utilisées dans les compositions selon l'invention ne comprennent pas de groupements ammoniums quaternaires.

Par milieu cosmétiquement acceptable, on entend un milieu compatible avec les fibres kératiniques, telles que les cheveux.

Le milieu cosmétiquement acceptable est constitué d'eau ou d'un mélange d'eau et d'un ou plusieurs solvants cosmétiquement acceptables choisis parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention varie généralement de 3 à 11 et de préférence de 5 à 10, mieux de 7 à 10.

La composition selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ , des céramides ; des esters gras huileux tels que le myristate d'isopropyle, le myristate de myristyle, le palmitate de cétyle et le stéarate de stéaryle ; des huiles minérales, végétales ou synthétiques telles que les α-oléfines ou l'huile d'avocat, l'huile de colza, l'huile d'abricot, l'huile de camélina, l'huile de vaseline ; des vitamines ou provitamines ; des polymères amphotères ; des agents de stabilisation du pH, des conservateurs ; et des colorants.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société CIBA, ACULYN 22, 28, 33, 44 ou 46 par la société ROHM & HAAS et ELFACOS T210 et T212 par la société AKZO.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

De préférence, les compositions cosmétiques de l'invention sont transparentes ou translucides, c'est-à-dire que ces compositions présentent une transmittance à 600 nanomètres supérieure à 85%, mieux supérieure à 90% et encore mieux supérieure à 94%.

Les compositions conformes à l'invention peuvent être utilisées comme shampooings pour le lavage et le conditionnement des cheveux et ils sont appliqués dans ce cas-là, de préférence, sur les cheveux humides dans des quantités efficaces pour les laver, et la mousse générée par massage ou friction avec les mains peut être ensuite éliminée après un éventuel temps de pause, par rinçage avec de l'eau, l'opération pouvant être répétée une ou plusieurs fois.

Un autre objet de l'invention est un procédé de traitement cosmétique des fibres kératiniques, telles que les cheveux, qui consiste à appliquer une quantité efficace d'une composition telle que décrite ci-dessus, sur lesdites fibres, et de préférence à rincer après un éventuel temps de pose.

En particulier, le procédé de traitement cosmétique des fibres kératiniques est un procédé de lavage et de conditionnement des fibres kératiniques, en particulier des cheveux.

L'exemple suivant sert à illustrer la présente invention.

### EXEMPLE

On prépare la composition (A) selon l'invention à partir des ingrédients indiqués dans le tableau ci-dessous dont les quantités sont exprimées en pour cent en poids en produit en l'état, par rapport au poids total de la composition.

| Composition | A (invention) |
|---|---|
| Acide lactique | 0,27 |
| Mélange de chloro-5-méthyl-2-iosthiazoline4-one-3 / méthylisothiazoline-4-one-3 / chlorure et nitrate de magnésium en solution aqueuse⁽¹⁾ | 0,1 |
| Oléate de propylène glycol polyéthoxylé (55 OE) et de propylène glycol en solution hydroglycolique ⁽²⁾ | 0,6 |
| Hydroxyéthyl cellulose quaternisée par le chlorure de 2,3 époxypropyl triméthyl ammonium à 95% M.A ⁽³⁾ | 0,6 |
| Polydiméthyl siloxane à groupements ⁽⁴⁾ aminoéthyl aminoisobutyle et triméthylsiloxy | 1 |
| 3-aminopropyltriéthoxysilane ⁽⁵⁾ | 0,75 |
| Cocyl bétaïne à 30% M.A en solution aqueuse⁽⁶⁾ | 17 |
| Alcool cétylique oxyéthyléné (20 OE) et oxypropyléné (5 OP) ⁽⁷⁾ | 0,5 |
| Acide lauryl éther carboxylique (4,5 OE) à 90% de matières actives dans l'eau ⁽⁸⁾ | 1 |
| Monoisopropanolamide d'acides de coprah à 94,5% de matières actives ⁽⁹⁾ | 0,85 |
| Lauryléther sulfate de sodium (2.2 OE) en solution aqueuse (70% M.A) ⁽¹⁰⁾ | 16 |
| Agent de mise au pH | qs pH=9 |
| Parfum | 0,5 |
| Eau désionisée | qsp 100 g |

| | |
|---|---|
| ⁽¹⁾ vendu sous la dénomination commerciale KATHON CG par la société ROHM et HAAS ⁽²⁾ vendu sous la dénomination commerciale ANTIL 141 LIQUID par la société EVONIK GOLDSCHMIDT ⁽³⁾ vendu sous la dénomination commerciale POLYQUTA 400 KC par la société KCI ⁽⁴⁾ vendu sous la dénomination commerciale DC 28566 AMINOFLUID par la société DOW CORNING ⁽⁵⁾ vendu sous la dénomination XIAMETER OFS EO11 SILANE par la société DOW CORNING ⁽⁶⁾ vendu sous la dénomination MIRATAINER BB/FLA par la société RHODIA, ⁽⁷⁾ vendu sous la dénomination PROCETYL A WS-LQ par la société CRODA, ⁽⁸⁾ vendu sous la dénomination AKYPO RLM 45 CA par la société KAO, ⁽⁹⁾ vendu sous la dénomination EMPILAN CIS par la société HUNTSMAN ⁽¹⁰⁾ vendu sous la dénomination Texapon AOS 225UP par la société COGNIS | |

On obtient une composition qui est limpide et stable dans le temps.

Appliquée en tant que shampooing, la composition (A) apporte des effets coiffants satisfaisants, en particulier cette composition confère aux cheveux de la masse, du volume ainsi qu'un toucher doux satisfaisant.

## Revendications

1. Composition cosmétique pour le lavage et le conditionnement des fibres kératiniques, en particulier des fibres kératiniques humaines telles que les cheveux, comprenant dans un milieu cosmétiquement acceptable :
(i) un ou plusieurs composés organiques du silicium choisis parmi les silanes comprenant un atome de silicium et les siloxanes comprenant deux ou trois atomes de silicium, lesdits composés organiques du silicium comportant en outre une ou plusieurs fonctions chimiques basiques et un ou plusieurs groupes hydroxyles ou hydrolysables par molécule ;
(ii) un ou plusieurs tensioactifs anioniques,
(iii) un ou plusieurs polymères cationiques choisis parmi :
(1)
(2) les celluloses cationiques,
et (4) les gommes de guar cationiques, et,
(iv) un ou plusieurs tensioactifs additionnels choisis parmi les tensioactifs amphotères et les tensioactifs non ioniques.

2. Composition cosmétique selon la revendication 1, **caractérisée en ce que** les fonctions chimiques basiques du composé organique du silicium sont choisies parmi les amines primaires, secondaires ou tertiaires.

3. Composition cosmétique selon la revendication 1 ou 2, **caractérisée en ce que** les groupes hydrolysables sont choisis parmi les groupes alcoxy, aryloxy et halogène.

4. Composition cosmétique selon l'une quelconque des revendications 1 à 3, **caractérisée en ce que** le ou les composés organiques du silicium sont choisis parmi les composés de formule (I) : 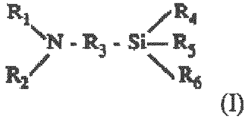 dans laquelle :
R₄ représente un halogène, un groupe OR' ou R'₁ ;
R₅ représente un halogène, un groupe OR" ou R'₂ ;
R₆ représente un halogène, un groupe OR''' ou R'₃ ;
R₁, R₂, R₃, R', R", R''', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R''' pouvant en outre désigner l'hydrogène, et deux au moins des groupes R₄, R₅ et R₆ désignant respectivement OR', OR" et OR''', deux au moins des groupes R', R" et R"' étant différents de l'hydrogène ; et 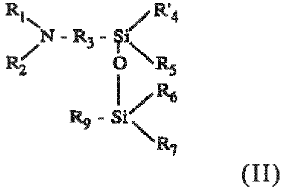 dans laquelle :
R₁, R₂, R₃, R₅ et R₆ sont définis comme précédemment ;
R'₄ représente un atome d'halogène ou un groupe OR₁₁ ;
R₇ représente un atome d'halogène, un groupe OR₁₀ ou R"₁ ;
R₉ représente un atome d'halogène, un groupe OR₈, R"₂ ou R₃NR₁R₂ ;
R"₁, R"₂, R₈, R₁₀ et R₁₁ représente un groupe hydrocarboné, saturé ou insaturé, linéaire ou ramifié, portant éventuellement des groupes chimiques supplémentaires, les groupes R₁₁, R₁₀ et R₈ pouvant en outre représenter un atome d'hydrogène ; l'un au moins des groupes R₆, R₇ et R₉ désignant un atome d'halogène, un groupe OR''', OR₁₀ ou OR₈.

5. Composition cosmétique selon la revendication 4, **caractérisée en ce que** les groupes R₁, R₂, R', R'₁, R'₂, R'₃, R", R''', R"₁, R"₂, R₈, R₁₀ et R₁₁ sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

6. Composition cosmétique selon l'une quelconque des revendications précédentes, **caractérisée en ce que** le ou les composés organiques du silicium sont choisis parmi les composés de formule (III) : 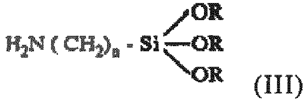 dans laquelle les radicaux R, identiques ou différents, sont choisis parmi les radicaux alkyle en C₁-C₆ et n est un nombre entier de 1 à 6, de préférence de 2 à 4.

7. Composition cosmétique selon l'une quelconque des revendications précédentes, **caractérisée en ce que** le ou les tensioactifs anioniques sont choisis parmi les alkylsulfates, les alkyléthersulfates et les alkyléthercarboxylates, et leurs mélanges, en particulier sous forme de sels de métaux alcalins ou alcalino-terreux, d'ammonium, d'amine ou d'aminoalcool.

8. Composition cosmétique selon l'une quelconque des revendications précédentes, **caractérisée en ce que** le ou les polymères cationiques sont choisis parmi les hydroxyalkylcelluloses, comme les hydroxyméthyl-, hydroxyéthyl- ou hydroxypropyl celluloses greffées notamment avec un sel de méthacryloyléthyltriméthylammonium, de méthacrylamidopropyltriméthylammonium, de diméthyldiallyl ammonium et les gommes de guar cationiques.

9. Composition cosmétique selon l'une quelconque des revendications précédentes, **caractérisée en ce qu'**elle présente un rapport pondéral entre le ou les composés organiques du silicium (i) et le ou les polymères cationiques (iii) allant de 0,1 à 20 et de préférence de 0,5 à 10, mieux de 0,8 à 5.

10. Composition cosmétique selon l'une quelconque des revendications précédentes, **caractérisée en ce qu'**elle comprend en outre une ou plusieurs silicones, de préférence aminées.

11. Procédé de traitement cosmétique des fibres kératiniques, en particulier des fibres kératiniques humaines telles que les cheveux, **caractérisée en ce que** l'on applique la composition cosmétique telle que définie selon l'une quelconque des revendications précédentes, sur lesdites fibres, et **en ce que** l'on rince après un éventuel temps de pose.

12. Utilisation d'une composition selon l'une quelconque des revendications 1 à 10, comme shampooing pour le nettoyage et le conditionnement des fibres kératiniques.

## Patentansprüche

1. Kosmetikzusammensetzung zum Waschen und Konditionieren von Keratinfasern, insbesondere menschlichen Keratinfasern, wie Haaren, die in einem kosmetisch annehmbaren Medium Folgendes umfasst:
(i) eine oder mehrere organische Siliziumverbindungen, ausgewählt aus Silanen, die ein Siliziumatom umfassen, und Siloxanen, die zwei oder drei Siliziumatome umfassen, wobei die organischen Siliziumverbindungen außerdem eine oder mehrere basische chemische Funktionen und eine oder mehrere Hydroxyl- oder hydrolysierbare Gruppen pro Molekül enthalten;
(ii) ein oder mehrere anionische Tenside,
(iii) ein oder mehrere kationische Polymere, ausgewählt aus:
(2) kationischen Cellulosen und
(4) kationischen Guargummen,
und
(iv) ein oder mehrere zusätzliche Tenside, ausgewählt aus amphoteren Tensiden und nicht-ionischen Tensiden.

2. Kosmetikzusammensetzung nach Anspruch 1, **dadurch gekennzeichnet, dass** die basischen chemischen Funktionen der organischen Siliziumverbindung aus primären, sekundären oder tertiären Aminen ausgewählt sind.

3. Kosmetikzusammensetzung nach Anspruch 1 oder 2, **dadurch gekennzeichnet, dass** die hydrolysierbaren Gruppen aus Alkoxy-, Aryloxy- und Halogengruppen ausgewählt sind.

4. Kosmetikzusammensetzung nach einem der Ansprüche 1 bis 3, **dadurch gekennzeichnet, dass** die organische(n) Siliziumverbindung(en) ausgewählt ist/sind aus Verbindungen der Formel (I): 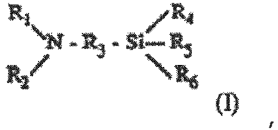 worin:
R₄ ein Halogen, eine Gruppe OR' oder R'₁ darstellt;
R₅ ein Halogen, eine Gruppe OR" oder R'₂ darstellt;
R₆ ein Halogen, eine Gruppe OR''' oder R'₃ darstellt;
R₁, R₂, R₃, R', R", R''', R'₁, R'₂, R'₃ unabhängig voneinander eine gesättigte oder ungesättigte, gerade oder verzweigte Kohlenwasserstoffgruppe darstellen, die gegebenenfalls weitere chemische Gruppen trägt, wobei R₁, R₂, R', R" und R''' auch für ein Wasserstoffatom stehen können, und mindestens zwei der Gruppen R₄, R₅ und R₆ jeweils für OR', OR" und OR''' stehen, wobei mindestens zwei der Gruppen R', R" und R'''
verschieden von Wasserstoff sind; und 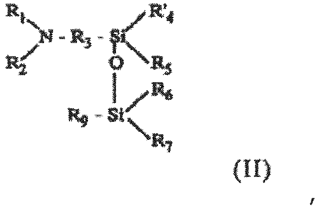 worin:
R₁, R₂, R₃, R₅ und R₆ wie vorstehend definiert sind;
R'₄ ein Halogenatom oder eine Gruppe OR₁₁ darstellt;
R₇ ein Halogenatom, eine Gruppe OR₁₀ oder R"₁ darstellt;
R₉ ein Halogenatom, eine Gruppe OR₈, R"₂ oder R₃NR₁R₂ darstellt;
R"₁, R"₂, R₈, R₁₀ und R₁₁ eine gesättigte oder ungesättigte, gerade oder verzweigte Kohlenwasserstoffgruppe darstellen, die gegebenenfalls weitere chemische Gruppen trägt, wobei die Gruppen R₁₁, R₁₀ und R₈ auch ein Wasserstoffatom darstellen können; wobei mindestens eine der Gruppen R₆, R₇ und R₉ für ein Halogenatom, eine Gruppe OR''', OR₁₀ oder OR₈ steht.

5. Kosmetikzusammensetzung nach Anspruch 4, **dadurch gekennzeichnet, dass** die Gruppen R₁, R₂, R', R'₁, R'₂, R'₃, R", R''', R"₁, R"₂, R₈, R₁₀ und R₁₁ aus C₁-C₁₂-Alkyl-, C₆- bis C₁₄-Aryl-, C₁- bis C₈-Alkyl-C₆- bis C₁₄-aryl- und C₆- bis C₁₄-Aryl-C₁- bis C₈-Alkylresten ausgewählt sind.

6. Kosmetikzusammensetzung nach einem der vorhergehenden Ansprüche, **dadurch gekennzeichnet, dass** die organische(n) Siliziumverbindung(en) aus den Verbindungen der Formel (III) ausgewählt ist/sind: 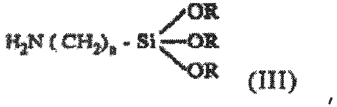 wobei die Reste R, die gleich oder verschieden sind, aus C₁-C₆-Alkylresten ausgewählt sind und n eine ganze Zahl von 1 bis 6, vorzugsweise von 2 bis 4 ist.

7. Kosmetikzusammensetzung nach einem der vorhergehenden Ansprüche, **dadurch gekennzeichnet, dass** das oder die anionische(n) Tensid(e) aus Alkylsulfaten, Alkylethersulfaten und Alkylethercarboxylaten und deren Gemischen, insbesondere in Form von Alkalimetall- oder Erdalkalimetall-, Ammonium-, Amin-, oder Aminoalkoholsalzen, ausgewählt ist/sind.

8. Kosmetikzusammensetzung nach einem der vorhergehenden Ansprüche, **dadurch gekennzeichnet, dass** das oder die kationische(n) Polymer(e) aus Hydroxyalkylcellulosen, wie Hydroxymethyl-, Hydroxyethyl- oder Hydroxypropylcellulosen, die insbesondere mit einem Methacryloylethyltrimethylammonium-, Methacrylamidopropyltrimethylammonium-, Dimethylallylammoniumsalz gepfropft sind, und kationischen Guargummen ausgewählt ist/sind.

9. Kosmetikzusammensetzung nach einem der vorhergehenden Ansprüche, **dadurch gekennzeichnet, dass** sie ein Gewichtsverhältnis zwischen der oder den organischen Siliziumverbindung(en) (i) und dem oder den kationischen Polymer(en) (iii) von 0,1 bis 20 und vorzugsweise von 0,5 bis 10, besser von 0,8 bis 5 aufweist.

10. Kosmetikzusammensetzung nach einem der vorhergehenden Ansprüche, **dadurch gekennzeichnet, dass** sie außerdem ein oder mehrere, vorzugsweise aminierte, Silikone umfasst.

11. Verfahren zur kosmetischen Behandlung von Keratinfasern, insbesondere menschlichen Keratinfasern, wie Haaren, **dadurch gekennzeichnet, dass** man die Kosmetikzusammensetzung nach einem der vorhergehenden Ansprüche auf die Fasern aufbringt und nach einer eventuellen Einwirkungsdauer ausspült.

12. Verwendung einer Zusammensetzung nach einem der Ansprüche 1 bis 10 als Shampoo zur Reinigung und Konditionierung von Keratinfasern.

## Claims

1. Cosmetic composition for washing and conditioning keratin fibres, in particular human keratin fibres such as the hair, comprising, in a cosmetically acceptable medium:
(i) one or more organosilicon compounds chosen from silanes comprising one silicon atom and siloxanes comprising two or three silicon atoms, the said organosilicon compounds also comprising one or more basic chemical functions and one or more hydroxyl or hydrolysable groups per molecule;
(ii) one or more anionic surfactants;
(iii) one or more cationic polymers chosen from:
(2) cationic celluloses, and
(4) cationic guar gums, and
(iv) one or more additional surfactants chosen from amphoteric surfactants and nonionic surfactant.

2. Cosmetic composition according to Claim 1, **characterized in that** the basic chemical functions of the organosilicon compound are chosen from primary, secondary and tertiary amines.

3. Cosmetic composition according to Claim 1 or 2, **characterized in that** the hydrolysable groups are chosen from alkoxy, aryloxy and halogen groups.

4. Cosmetic composition according to any one of Claims 1 to 3, **characterized in that** the organosilicon compounds(s) are chosen from the compounds of formula (I) : 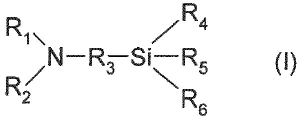 in which:
R₄ represents a halogen or a group OR' or R'₁;
R₅ represents a halogen or a group OR" or R'₂;
R₆ represents a halogen or a group OR''' or R'₃;
R₁, R₂, R₃, R', R", R''', R'₁, R'₂ and R'₃ represent, independently of each other, a saturated or unsaturated, linear or branched hydrocarbon-based group optionally bearing additional chemical groups, R₁, R₂, R', R" and R''' also possibly denoting hydrogen, and at least two of the groups R₄, R₅ and R₆ respectively denoting OR', OR" and OR''', at least two of the groups R' , R" and R''' being other than hydrogen; and 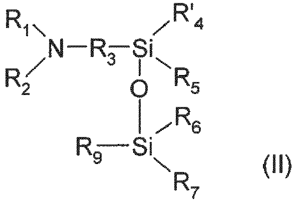 in which:
R₁, R₂, R₃, R₅ and R₆ are as defined previously;
R'₄ represents a halogen atom or a group OR₁₁;
R₇ represents a halogen atom or a group OR₁₀ or R"₁;
R₉ represents a halogen atom or a group OR₈, R"₂ or R₃NR₁R₂;
R"₁, R"₂, R₈, R₁₀ and R₁₁ represent a saturated or unsaturated, linear or branched hydrocarbon-based group, optionally bearing additional chemical groups, R₁₁, R₁₀ and R₈ also possibly representing a hydrogen atom; at least one of the groups R₆, R₇ and R₉ denoting a halogen atom or a group OR''', OR₁₀ or OR₈.

5. Cosmetic composition according to Claim 4, **characterized in that** the groups R₁, R₂, R', R'₁, R'₂, R'₃, R", R''', R"₁, R"₂, R₈, R₁₀ and R₁₁ are chosen from C₁-C₁₂ alkyl, C₆-C₁₄ aryl, (C₁-C₈) alkyl (C₆-C₁₄) aryl and (C₆-C₁₄) aryl (C₁-C₈) alkyl radicals.

6. Cosmetic composition according to any one of the preceding claims, **characterized in that** the organosilicon compound(s) are chosen from the compounds of formula (III): 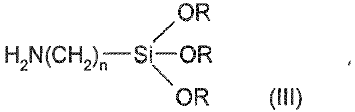 in which the radicals R, which may be identical or different, are chosen from C₁-C₆ alkyl radicals, and n is an integer from 1 to 6 and preferably from 2 to 4.

7. Cosmetic composition according to any one of the preceding claims, **characterized in that** the anionic surfactant(s) are chosen from alkyl sulfates, alkyl ether sulfates and alkyl ether carboxylates, and mixtures thereof, in particular in the form of alkali metal or alkaline-earth metal, ammonium, amine or amino alcohol salts.

8. Cosmetic composition according to any one of the preceding claims, **characterized in that** the cationic polymer(s) are chosen from hydroxyalkylcelluloses, such as hydroxymethyl-, hydroxyethyl- or hydroxypropyl- celluloses grafted especially with a methacryloylethyltrimethylammonium, methylacrylamidopropyl¬trimethylammonium or dimethyldiallylammonium salt, and cationic guar gums.

9. Cosmetic composition according to any one of the preceding claims, **characterized in that** it has a weight ratio between the organosilicon compound(s) (i) and the cationic polymer(s) (iii) ranging from 0.1 to 20, preferably from 0.5 to 10 and better still from 0.8 to 5.

10. Cosmetic composition according to any one of the preceding claims, **characterized in that** it also comprises one or more silicones, preferably amino silicones.

11. Cosmetic process for treating keratin fibres, in particular human keratin fibres such as the hair, **characterized in that** the cosmetic composition as defined according to any one of the preceding claims is applied to the said fibres, and **in that** it is rinsed out after an optional leave-on time.

12. Use of a composition according to any one of Claims 1 to 10, as a shampoo for cleansing and conditioning keratin fibres.
